Europäisches Patentamt

(19) **European Patent Office**

**Office européen des brevets**

(11) Numéro de publication: **0 027 986**
**B1**

(12) **FASCICULE DE BREVET EUROPEEN**

(45) Date de publication du fascicule du brevet:
**30.05.84**

(21) Numéro de dépôt: **80106373.6**

(22) Date de dépôt: **20.10.80**

(51) Int. Cl.³: **G 01 N 21/07,** G 01 N 1/00,
G 01 N 33/54

(54) **Dispositif d'analyses simultanées.**

(30) Priorité: **26.10.79 FR 7926615**
**11.01.80 FR 8000583**
**23.05.80 FR 8011511**

(43) Date de publication de la demande:
**06.05.81 Bulletin 81/18**

(45) Mention de la délivrance du brevet:
**30.05.84 Bulletin 84/22**

(84) Etats contractants désignés:
**AT BE CH DE FR GB IT LI LU NL SE**

(56) Documents cités:
**FR - A - 1 481 289**
**FR - A - 2 280 895**
**FR - A - 2 362 660**
**FR - A - 2 378 283**
**FR - A - 2 400 700**
**FR - A - 2 418 463**
**FR - A - 2 424 531**
**GB - A - 2 017 910**
**US - A - 4 053 284**
**US - A - 4 116 638**

(73) Titulaire: **Guigan, Jean, 9, rue Jean Mermoz,**
**F-75008 Paris (FR)**

(72) Inventeur: **Guigan, Jean, 9, rue Jean Mermoz,**
**F-75008 Paris (FR)**

(74) Mandataire: **Weinmiller, Jürgen et al, Zeppelinstrasse 63,**
**D-8000 München 80 (DE)**

ACTORUM AG

## Description

L'invention concerne un dispositif d'analyses simultanées utilisant un support solide d'accrochage destiné à capter successivement une quantité d'un premier réactif tel qu'un liquide biologique (sérum ou plasma par exemple) contenant la substance à analyser, puis une quantité d'un deuxième réactif contenant une protéine sur laquelle est fixé un indicateur biologique (par exemple un enzyme, ou un radio-isotope pour la radio-immunologie).

Les techniques actuelles utilisent un support solide d'accrochage qui est usuellement une bille sphérique en polystyrène recouvert d'une protéine ayant des propriétés de type anti-corps (par exemple un polypeptide provenant d'un animal vacciné). Ce support est disposé dans un récipient de type tube à essais dans lequel est alors admise une quantité d'un premier réactif, et, après un temps d'incubation, il faut procéder à un lavage énergique à l'eau de la bille pour enlever l'excès de réactif, lequel lavage est opéré manuellement ainsi que l'évacuation du liquide en retournant le tube dont l'extrémité ouverte présente des pattes évitant l'éjection de la bille; cette opération doit être renouvelée avec le deuxième réactif dont l'excès est de nouveau évacué par lavage, après quoi le tube est transporté vers un poste d'analyse proprement dite, par exemple pour une analyse photocolorimétrique en injectant un réactif coloré.

Autant le principe de la bille d'accrochage captant successivement la quantité prévue de réactif est simple et performant, autant la technique utilisée dans l'art antérieur donne peu de satisfactions: en effet, les opérations de lavage effectuées manuellement sont peu fiables en raison de l'hétérogénéité des lavages et des conditions de température, et sont impropres à une analyse par tests groupés avec des dosages différents en raison des nombreuses manipulations qui sont nécessaires et des risques d'erreurs inévitables.

La présente invention a pour but de proposer un dispositif plus fiable, de conception simple, et parfaitement adapté aux analyses simultanées correspondant à plusieurs dosages, avec le minimum de manipulations.

Ce but est atteint conformément à l'invention par un dispositif d'analyses simultanées utilisant un support solide d'accrochage destiné à capter successivement une quantité d'un premier réactif tel qu'un liquide biologique contenant la substance à analyser, puis une quantité d'un deuxième réactif contenant une protéine sur laquelle est fixé un indicateur biologique, caractérisé par le fait qu'il est essentiellement constitué d'un rotor d'analyse comportant d'une part une pluralité de cellules périphériques contenant chacune ledit support d'accrochage, et d'autre part des moyens permettant l'acheminement d'un liquide de lavage vers chaque cellule, chacune desdites cellules étant munie d'un orifice périphérique d'éjection de liquide, et présentant une portion supérieure munie d'un orifice d'admission de réactif et une portion inférieure recevant ledit réactif.

Le dispositif d'analyses simultanées de l'invention peut présenter en outre l'une au moins des caractéristiques suivantes:

— les moyens permettant l'acheminement d'un liquide de lavage sont constitués par un orifice central d'admission duquel partent des conduits radiaux reliant ledit orifice central à chacune des cellules périphériques; de préférence, les conduits radiaux sont dans un plan perpendiculaire à l'axe de rotation du dispositif, et débouchent dans chaque cellule sensiblement au même niveau que l'orifice périphérique d'éjection de liquide de ladite cellule,

— les orifices d'admission d'un réactif sont équidistants de l'axe du rotor,

— les parois définissant la portion inférieure de chaque cellule permettent des analyses photométriques simultanées,

— le rotor est obtenu par superposition de deux portions coaxiales dont les bords périphériques en contact définissent directement les orifices périphériques des cellules,

— le support d'accrochage est formé par au moins une bille,

— le fond de chaque cellule présente une allure générale inclinée déterminée de telle façon que d'une part la portion inférieure de la cellule présente un volume suffisant pour recevoir la bille et la quantité de réactif la recouvrant, et que d'autre part ladite bille soit guidée sensiblement jusqu'au niveau de l'orifice périphérique de ladite cellule lors de la centrifugation,

— le support est constitué par une pluralité de billes de très faibles dimensions; une barrière est disposée dans chaque cellule en regard de l'orifice périphérique d'éjection, ladite barrière étant apte à retenir des billes au cours de la centrifugation;

— les faces interne et externe des cellules sont sensiblement parallèles, la face externe présentant en outre un déversoir latéral terminé par l'orifice d'éjection et permettant l'évacuation totale du liquide de lavage lors de la centrifugation,

— le support d'accrochage est constitué par la paroi de la portion inférieure de chaque cellule.

D'autres caractéristiques et avantages d'une mise en œuvre préférée de l'invention apparaîtront plus clairement à la lumière de la description qui va suivre, donnée à titre illustratif mais nullement limitatif, en rérérence aux figures du dessin annexé, où:

— la fig. 1 est une vue partielle en perspective illustrant un dispositif d'analyses simultanées conforme à l'invention,

— la fig. 2 est une coupe axiale partielle montrant l'obtention du dispositif de la fig. 1,

— la fig. 3 est une vue de dessus partielle du même dispositif,

— les figs 4A, 4B, 4C illustrent le fonctionnement du dispositif selon la fig. 1,

— les figs 5A et 5B illustrent le fonctionnement du dispositif dans le cas où la cellule contient une pluralité de petites billes,

— les figs 6 et 7 illustrent le fonctionnement du dispositif dans le cas où le support d'accrochage

est formé par la paroi de la portion inférieure de la cellule.

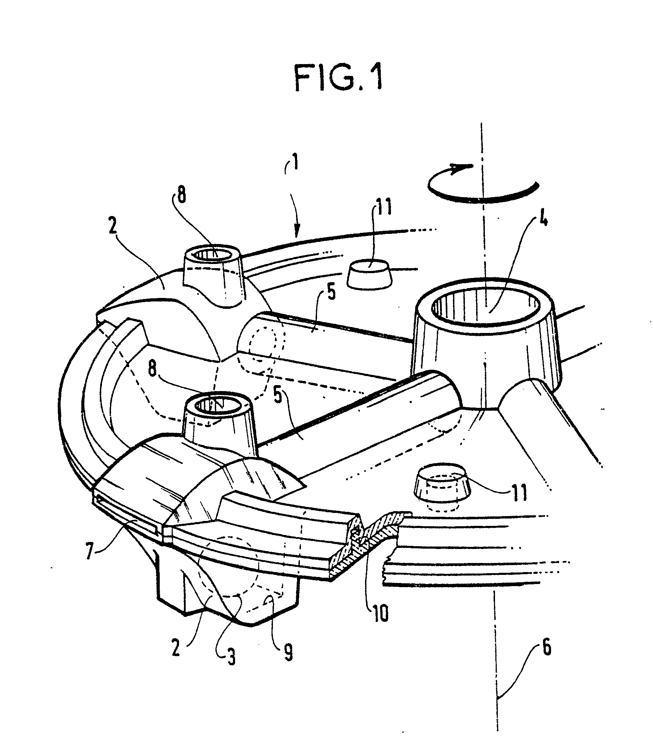

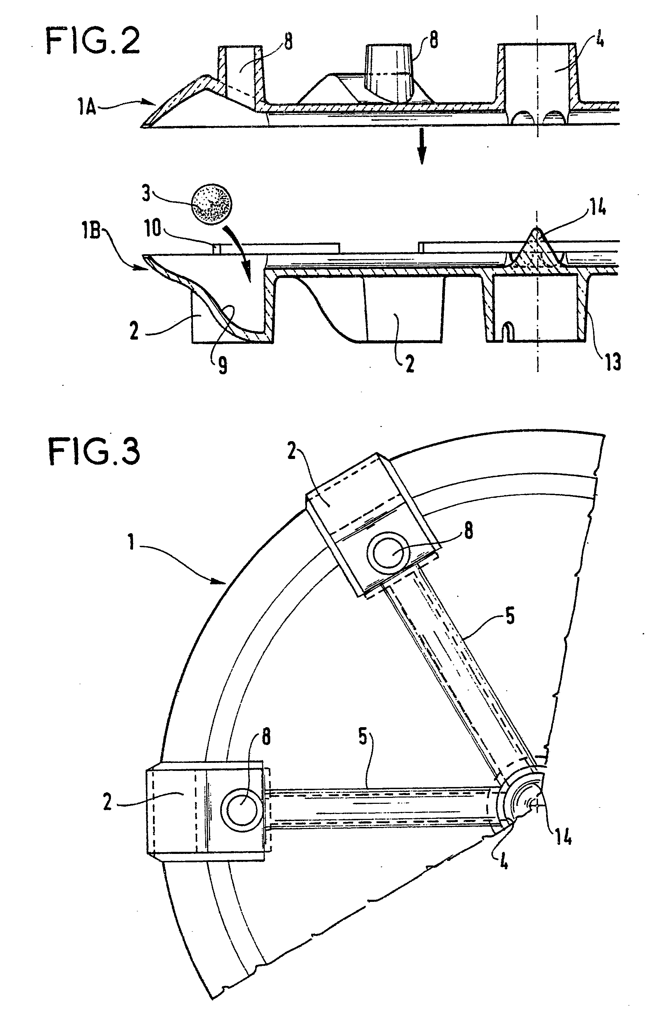

Figs 1 à 3, le dispositif d'analyses simultanées illustré, conforme à l'invention, est essentiellement constitué d'un rotor 1 comportant d'une part une pluralité de cellules périphériques 2 contenant chacune une bille d'accrochage 3 classique (en polystyrène recouvert d'une protéine ayant des propriétés de type anti-corps), et d'autre part des moyens permettant l'acheminement d'un liquide de lavage vers chaque cellule. Plusieurs moyens sont possibles pour acheminer un liquide de lavage: on a illustré ici une possibilité particulièrement simple et efficace en prévoyant un orifice central d'admission 4 duquel partent des conduits radiaux 5 reliant ledit orifice central à chaque cellule périphérique 2, lesdits conduits étant dans un plan perpendiculaire à l'axe de rotation 6 du dispositif; on pourrait aussi bien prévoir une gouttière annulaire à la place de l'orifice central 4, de laquelle partiraient des conduits de raccordement radiaux vers chaque cellule périphérique. Il est à noter que les conduits radiaux 5 sont ici définis par deux portions complémentaires formées sur chacune des deux parties 1A, 1B formant le rotor 1 (voir fig. 2), mais que tout autre type de conduit serait envisageable, tel par exemple qu'un bord plat sur une partie du rotor (donc sa face interne) et une rainure sur l'autre partie.

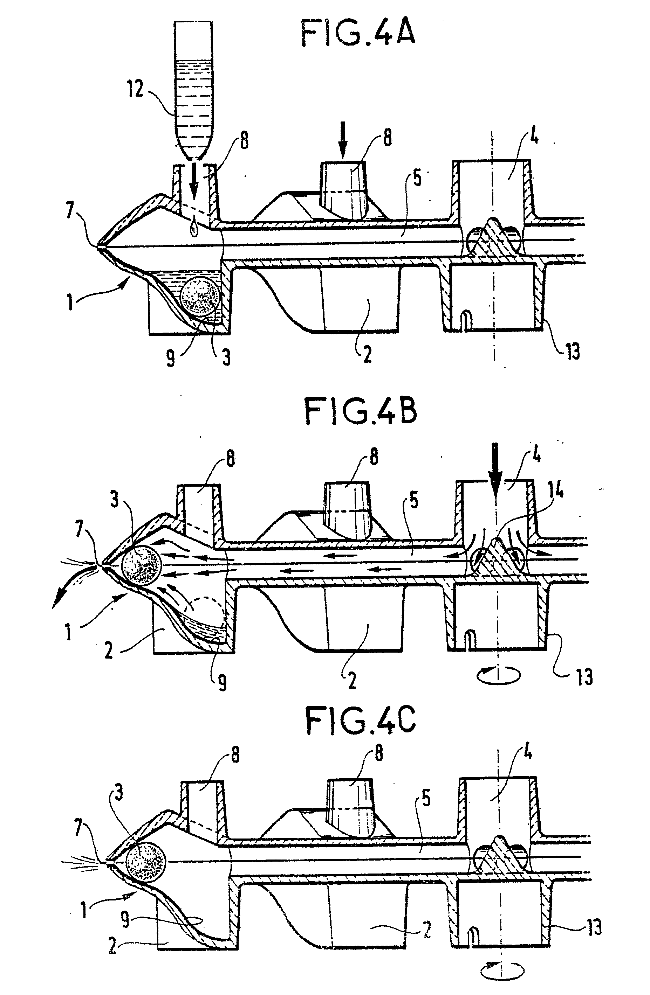

Chaque cellule 2 est par ailleurs munie d'un orifice périphérique 7 d'éjection de liquide, ici défini directement par superposition des bords périphériques des deux portions coaxiales 1A, 1B formant le rotor 1, et présente une portion supérieure munie d'un orifice 8 d'admission et dont le fond 9 permet de remonter par centrifugation la bille 3 sensiblement au niveau dudit orifice périphérique 7 lorsque le dispositif est mis en rotation (cette dernière caractéristique sera expliquée plus loin au regard des figs 4A, 4B, 4C).

L'homme de l'art choisira tout moyen pour la liaison et le positionnement respectif des deux portions 1A, 1B formant le rotor 1: on a illustré ici à titre d'exemple l'emploi d'une rainure annulaire 10 et de tétons de centrage 11, mais de nombreuses autres possibilités sont envisageables.

Les caractéristiques du dispositif d'analyses simultanées concernant en particulier la forme des cellules vont être décrites en même temps que le fonctionnement dudit dispositif, afin de mieux percevoir la fonction qu'elles remplissent:

– fig. 4A, le rotor 1 dont chaque cellule périphérique 2 est munie de sa bille associée 3 (chaque bille pouvant correspondre à un dosage particulier) est disposé sous un dispositif d'alimentation, ici une pipette 12, de façon à injecter dans chaque cellule par son orifice 8, un volume (de préférence calibré) d'un premier réactif tel que sérum, plasma ou tout autre liquide biologique contenant la substance à analyser; après cette injection, qui peut être obtenue par exemple en faisant successivement tourner le rotor de l'angle correspondant pour placer chaque orifice 8 sous la pipette 12, on laisse reposer pendant le temps d'incubation nécessaire. Il est à noter que le fond 9 de chaque cellule présente un volume suffisant pour recevoir la bille et le réactif sans que le niveau de celui-ci atteigne les conduits 5,

– fig. 4B, le rotor 1 est mis en rotation par des moyens d'entraînement saillants 13 tout à fait classiques, et on injecte du liquide de lavage par l'orifice central 4: pour chaque cellule, la centrifugation a pour effet d'éjecter l'excès de réactif par l'orifice périphérique 7, et de déplacer la bille 3 jusqu'au niveau dudit orifice périphérique grâce au fond 9 d'allure générale inclinée. En outre, le liquide de lavage, dont la bonne répartition est favorisée par la présence d'une protubérance centrale 14, passe par les conduits radiaux 5, et vient laver énergiquement chaque bille 3 dont la position est tout à fait favorable sous l'effet de la centrifugation, grâce en particulier à la forme affinée vers la périphérie pour chaque cellule, ledit liquide s'échappant par les orifices périphériques 7 après avoir entouré chaque bille (en la faisant d'ailleurs tourner sur elle-même),

– fig. 4C, il s'agit d'une phase facultative de séchage des billes, obtenue en coupant l'admission du liquide de lavage tout en maintenant la centrifugation. Il est à noter que ce séchage était pratiquement impossible à obtenir avec les dispositifs antérieurs, à moins de montages compliqués, alors qu'ici il est obtenu avec une facilité extrême.

Ces opérations sont ensuite réitérées avec le deuxième réactif, qui contient une protéine sur laquelle est fixé un indicateur biologique tel par exemple qu'un enzyme ou un radio-isotope. Alors, une fois le rotor arrêté, les billes de chaque cellule redescendent et sont prêtes à servir pour des analyses simultanées (les parois définissant la portion inférieure des cellules étant prévues pour de telles analyses), analyses directes avec un compteur si l'on utilise des radio-isotopes, ou indirectes si l'on utilise en plus un réactif coloré et éventuellement un réactif de stoppage de réaction.

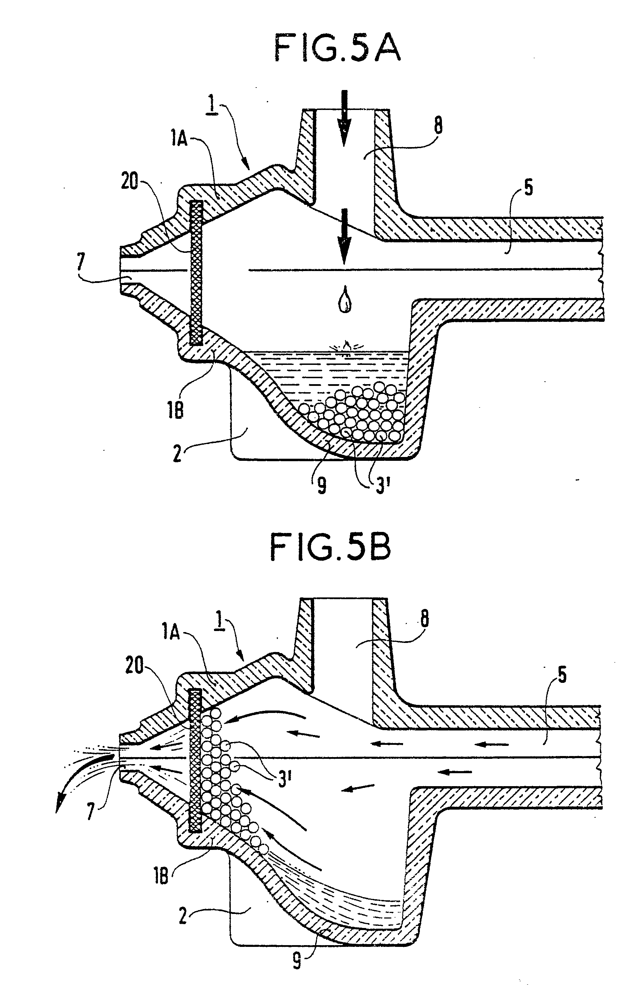

Les figs 5A et 5B correspondent au cas où chaque cellule périphérique 2 du rotor 1 comporte non plus une seule bille mais une pluralité de petites billes 3′, dont le diamètre est de l'ordre de 10 à 20 microns. Par ailleurs, la référence 20 désigne une barrière apte à retenir les billes au cours de la rotation du dispositif, cette barrière étant encastrée ou collée dans des échancrures aménagées dans les portions 1A et 1B formant le rotor 1. Avantageusement, une telle barrière est du type filtre moléculaire.

En conséquence chaque cellule périphérique 2 munie de ses billes associées 3′ (les billes de chaque cellule pouvant correspondre à un dosage particulier) est disposée sous un dispositif d'alimentation.

Sur la fig. 5B, qui correspond au moment où le rotor est en rotation et où on injecte le liquide de lavage, on peut constater que la centrifugation a pour effet, d'éjecter l'excès de réactif par l'orifice périphérique 7 et de déplacer les billes 3′ jusqu'à ce qu'elles soient arrêtées par la barrière 20 grâce au fond 9 d'allure générale inclinée.

On doit noter que la présence de nombreuses billes de faible diamètre permet d'augmenter notablement la surface d'accrochage du réactif et la fiabilité.

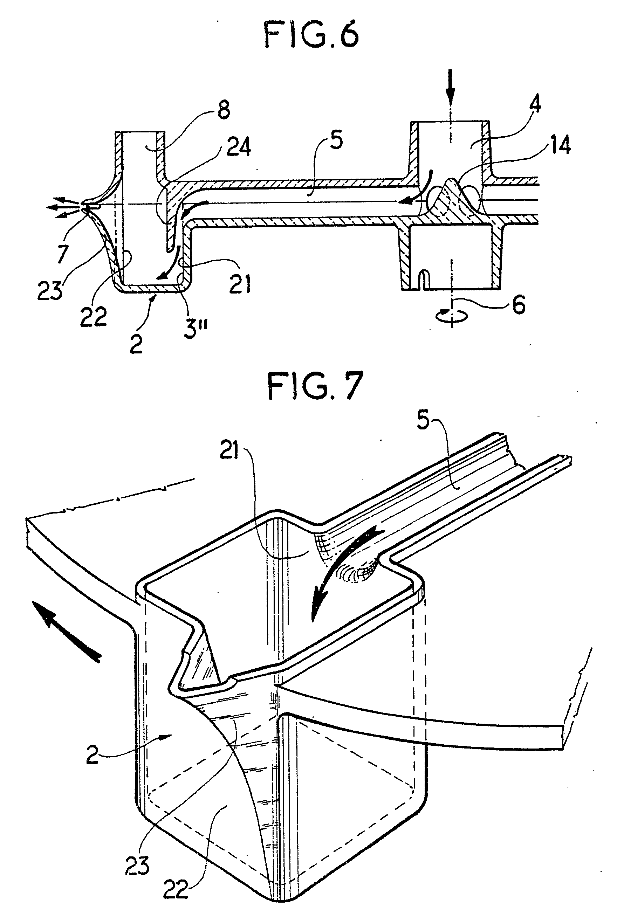

Les cellules périphériques d'analyse peuvent avoir une autre forme que celle représentée aux figs 1 à 5B. En particulier, elles peuvent avoir la forme représentée aux figs 6 et 7, où les faces interne et externe sont sensiblement parallèles et où la face externe présente un déversoir latéral terminé par l'orifice d'éjection.

Les figs 6 et 7 se rapportent au cas où le support d'accrochage est formé par la paroi de la position inférieure de la cellule, mais la forme générale de la cellule peut être utilisée lorsque le support est constitué d'une ou plusieurs billes.

Le dispositif d'analyses simultanées présente la même configuration générale que précédemment.

Le support solide d'accrochage est ici constitué par la paroi de la portion inférieure 3″ des cellules 2 qui est recouverte d'une protéine ayant des propriétés de type anti-corps. Les faces interne 21 et externe 22 de la cellule au niveau de cette portion inférieure sont parallèles. De plus la face externe 22 présente un déversoir latéral 23 terminé par l'orifice d'éjection 7, ce déversoir étant positionné, comme représenté, à la partie postérieure de la cellule par rapport au sens de rotation.

Le rotor est disposé sous un dispositif d'alimentation, de façon à injecter dans chaque cellule 2 par son orifice 8, un volume (de préférence calibré) d'un premier réactif tel que sérum, plasma ou tout autre liquide biologique contenant la substance à analyser; après cette injection, on laisse reposer pendant le temps d'incubation nécessaire.

Le rotor est ensuite mis en rotation et on injecte du liquide de lavage par l'orifice central 4. A la sortie des conduits 5, on prévoit des moyens 24 formant chicanes de manière à bien diriger le liquide de lavage vers le fond de la cellule. Pour chaque cellule, la centrifugation a pour effet d'éjecter l'excès de réactif par l'orifice périphérique 7. En outre, le liquide de lavage, dont la bonne répartition est favorisée par la présence d'une protubérance centrale 14, passe par les conduits radiaux 5, et vient laver énergiquement les parois de la portion inférieure de chaque cellule 2, ledit liquide s'échappant par les orifices périphériques 7, l'évacuation étant complète du fait de la présence du déversoir latéral 23.

Après le lavage on peut procéder facultativement à un séchage en coupant l'admission du liquide de lavage tout en maintenant la centrifugation.

Ces opérations sont ensuite réitérées avec le deuxième réactif, qui contient une protéine sur laquelle est fixé un indicateur biologique tel par exemple qu'un enzyme ou un radio-isotope. Une fois le rotor arrêté, on peut procéder à des analyses simultanées, analyses directes avec un compteur si l'on utilise des radio-isotopes, ou indirectes si l'on utilise en plus un réactif coloré et éventuellement un réactif de stoppage de réaction.

Le parallélisme des faces interne et externe de la portion inférieure des cellules facilite bien évidemment les analyses photométriques au niveau de chaque cellule.

Toutefois, dans le cas où le support solide d'accrochage est constitué par la paroi de la portion inférieure 3″ des cellules périphériques 2, ces dernières peuvent présenter une autre forme, en particulier celle correspondant aux figs 1 à 5B.

Le fonctionnement du dispositif selon l'invention met en évidence la grande simplicité obtenue, ainsi que la fiabilité élevée puisque les traitements sont très homogènes, tout en nécessitant peu de manipulations. A titre d'exemple, le dispositif de l'invention permet d'obtenir rapidement et simplement les cinq dosages simultanés requis pour une analyse de thyroïde. La souplesse est aussi illustrée par la possibilité parmi d'autres de diviser le rotor en deux zones pour utiliser certaines cellules comme étalons de contrôle, et de prévoir soit des supports d'accrochage identiques, ce qui permet d'effectuer un même test pour plusieurs clients, soit des supports différents, ce qui permet notamment d'effectuer plusieurs tests pour un même client.

Mentionnons enfin l'avantage considérable que représente le faible encombrement, puisqu'un tel dispositif peut être réalisé jusqu'à des diamètres de l'ordre de 5 cm.

**Revendications**

1. Dispositif d'analyses simultanées utilisant un support solide d'accrochage (3, 3', 3″) destiné à capter successivement une quantité d'un premier réactif tel qu'un liquide biologique contenant la substance à analyser, puis une quantité d'un deuxième réactif contenant une protéine sur laquelle est fixé un indicateur biologique, caractérisé par le fait qu'il est essentiellement constitué d'un rotor d'analyse (1) comportant d'une part une pluralité de cellules périphériques (2) contenant chacune ledit support d'accrochage (3, 3', 3″), et d'autre part des moyens (4, 5) permettant l'acheminement d'un liquide de lavage vers chaque cellule, chacune desdites cellules (2) étant munie d'un orifice périphérique d'éjection (7) de liquide, et présentant une portion supérieure munie d'un orifice d'admission (8) de réactif et une portion inférieure recevant ledit réactif.

2. Dispositif d'analyses simultanées selon la revendication 1, caractérisé par le fait que les moyens permettant l'acheminement du liquide de lavage sont constitués par un orifice central d'admission (4) duquel partent des conduits radiaux (5) reliant ledit orifice central (4) à chacune des cellules périphériques (2).

3. Dispositif d'analyses simultanées selon la revendication 2, caractérisé par le fait que les conduits radiaux (5) sont dans un plan perpendiculaire à l'axe de rotation du dispositif.

4. Dispositif d'analyses simultanées selon l'une des revendications 2 et 3, caractérisé par le fait que les conduits radiaux (5) débouchent dans chaque cellule sensiblement au même niveau que l'orifice périphérique d'éjection (7) de liquide de ladite cellule.

5. Dispositif d'analyses simultanées selon l'une des revendications précédentes, caractérisé par le fait que les orifices d'admission (8) d'un réactif sont équidistants de l'axe du rotor.

6. Dispositif d'analyses simultanées selon l'une des revendications précédentes, caractérisé par le fait que les parois définissant la portion inférieure de chaque cellule (2) permettent des analyses photométriques simultanées.

7. Dispositif d'analyses simultanées selon l'une des revendications précédentes, caractérisé par le fait que le rotor est obtenu par superposition de deux portions coaxiales (1A, 1B) dont les bords périphériques en contact définissent directement les orifices périphériques (7) des cellules.

8. Dispositif d'analyses simultanées selon l'une des revendications précédentes, caractérisé par le fait que le support d'accrochage est formé par au moins une bille (3).

9. Dispositif d'analyses simultanées selon la revendication 8, caractérisé par le fait que le support étant constitué par une pluralité de billes (3') de très faibles dimensions, une barrière (20) est disposée dans chaque cellule en regard dudit orifice périphérique d'éjection (7), ladite barrière (20) étant apte à retenir les billes (3') au cours de ladite centrifugation.

10. Dispositif d'analyses simultanées selon la revendication 9, caractérisé par le fait que ladite barrière (20) est du type filtre moléculaire.

11. Dispositif d'analyses simultanées selon l'une des revendications 9 et 10, caractérisé par le fait que lesdites billes (3') présentent un diamètre compris entre 10 et 20 microns.

12. Dispositif d'analyses simultanées selon l'une des revendications 1 à 7, caractérisé par le fait que le support d'accrochage (3'') est constitué par la paroi de la portion inférieure de chaque cellule (2).

13. Dispositif d'analyses simultanées selon l'une des revendications 8 à 11, caractérisé par le fait que le fond (9) de chaque cellule (2) présente une allure générale inclinée déterminée de telle façon que, d'une part, la portion inférieure de la cellule présente un volume suffisant pour recevoir le support d'accrochage et la quantité de réactif le recouvrant, et que, d'autre part, ledit support soit guidé sensiblement jusqu'au niveau de l'orifice périphérique (7) de ladite cellule (2) lors de la centrifugation.

14. Dispositif d'analyses simultanées selon l'une des revendications 1 à 12, caractérisé par le fait que les faces interne (21) et externe (22) des cellules sont sensiblement parallèles, la face externe (22) présentant en outre un déversoir latéral (23) terminé par l'orifice d'éjection (7) et permettant l'évacuation totale du liquide de lavage lors de la centrifugation.

15. Dispositif d'analyses simultanées selon la revendication 14, caractérisé par le fait que ledit déversoir (23) est positionné à la partie postérieure de la cellule (2) par rapport au sens de rotation.

## Claims

1. A device for simultaneous analysis utilizing a solid adhesion support (3, 3', 3'') destined to hold successively a quantity of a first reagent such as a biological liquid containing the substance to be analysed, then a quantity of a second reagent containing a protein on which a biological indicator is fixed, characterized in that it is essentially constituted by an analysis rotor (1) comprising on the one hand a plurality of peripheral cells (2) containing each said adhesion support (3, 3', 3''), and on the other hand means (4, 5) adapted to convey a washing liquid towards each cell, each of said cells (2) being provided with a peripheral orifice (7) for removing the liquid, and presenting an upper portion provided with an admission orifice (8) for the reagent and a lower portion receiving said reagent.

2. A device for simultaneous analysis according to claim 1, characterized in that the means adapted to convey the washing liquid are constituted by a central admission orifice (4) from which radial ducts (5) originate to connect said central orifice (4) to each peripheral cell (2).

3. A device for simultaneous analysis according to claim 2, characterized in that the radial ducts (5) are in a plane which is perpendicular to the rotation axis of the device.

4. A device for simultaneous analysis according to one of the claims 2 and 3, characterized in that the radial ducts (5) each communicate with a cell sensibly at the same level as the peripheral orifice (7) for removing the liquid from said cell.

5. A device for simultaneous analysis according to one of the preceding claims, characterized in that the admission orifices (8) of a reagent are equidistant from the axis of the rotor.

6. A device for simultaneous analysis according to one of the preceding claims, characterized in that the walls defining the lower portion of each cell (2) allow simultaneous photometric analyses.

7. A device for simultaneous analysis according to one of the preceding claims, characterized in that the rotor is obtained by superposing two coaxial portions (1A, 1B), the peripheral contacting edges of which define directly the peripheral orifices (7) of the cells.

8. A device for simultaneous analysis according to one of the preceding claims, characterized in that the adhesion support is formed by at least one bead (3).

9. A device for simultaneous analysis according to claim 8, characterized in that, the support being constituted by a plurality of beads (3') of very small dimensions, a barrier (20) is disposed in each cell adjacent said peripheral removal orifice (7), said barrier (20) being adapted to retain the beads (3') during said centrifuging operations.

10. A device for simultaneous analysis according to claim 9, characterized in that said barrier is of the molecular filter type.

11. A device for simultaneous analysis according to one of the claims 9 and 10, characterized in that the diameter of said beads (3') lies between 10 and 20 µm.

12. A device for simultaneous analysis according to one of the claims 1 to 7, characterized in that the adhesion support (3'') is constituted by the wall of the lower portion of each cell (2).

13. A device for simultaneous analysis according to one of the claims 8 to 11, characterized in that the bottom (9) of each cell (2) is generally inclined so that on the one hand, the lower portion of the cell presents a volume which is sufficient to accommodate the adhesion support and the quantity of reagent which covers it, and that, on the other hand, said support is guided sensibly up to the level of the peripheral orifice (7) of said cell (2) during the centrifuging operation.

14. A device for simultaneous analysis according to one of the claims 1 to 12, characterized in that the inner (21) and outer faces (22) of the cells are sensibly parallel, the outer face (22) further presenting a lateral spout (23) ending in the removal orifice (7) and being suitable to totally evacuate the washing liquid during the centrifuging operation.

15. A device for simultaneous analysis according to claim 14, characterized in that said spout (23) is positioned at the rear of the cell (2) with respect to the rotation direction.


**Patentansprüche**

1. Vorrichtung zur Simultananalyse, die einen festen Haftträger (3, 3', 3'') verwendet, der dazu bestimmt ist, nacheinander eine Menge eines ersten Reagenzstoffes wie z.B. einer biologischen Flüssigkeit, die die zu analysierende Substanz enthält, und dann einer Menge eines zweiten Reagenzstoffes festzuhalten, der ein Protein enthält, an dem ein biologischer Indikator befestigt ist, dadurch gekennzeichnet, dass sie im wesentlichen aus einem Analyserotor (1) besteht, der einerseits eine Vielzahl von peripheren Zellen (2), die jede den Haftträger (3, 3', 3'') enthalten, und anderseits Mittel (4, 5) aufweist, die den Transport einer Waschflüssigkeit zu jeder Zelle ermöglichen, wobei jede dieser Zellen (2) mit einer peripheren Öffnung (7) zur Entfernung der Flüssigkeit versehen ist und einen oberen Teil, der mit einer Zufuhröffnung (8) für den Reagenzstoff versehen ist, und einen unteren Teil aufweist, der diesen Reagenzstoff enthält.

2. Vorrichtung zur Simultananalyse nach Anspruch 1, dadurch gekennzeichnet, dass die Mittel, die den Transport einer Waschflüssigkeit ermöglichen, aus einer zentralen Zufuhröffnung (4) bestehen, von der radiale Leitungen (5) ausgehen, die die zentrale Öffnung (4) mit jeder der peripheren Zellen (2) verbinden.

3. Vorrichtung zur Simultananalyse nach Anspruch 2, dadurch gekennzeichnet, dass die radialen Leitungen (5) sich in einer zur Rotationsachse der Vorrichtung senkrechten Ebene befinden.

4. Vorrichtung zur Simultananalyse nach einem der Ansprüche 2 und 3, dadurch gekennzeichnet, dass die radialen Leitungen (5) in jeder Zelle im wesentlichen in der gleichen Höhe wie die periphere Öffnung (7) zur Entfernung der Flüssigkeit aus der Zelle münden.

5. Vorrichtung zur Simultananalyse nach einem der vorstehenden Ansprüche, dadurch gekennzeichnet, dass die Zufuhröffnungen (8) für einen Reagenzstoff gleiche Abstände zur Rotorachse haben.

6. Vorrichtung zur Simultananalyse nach einem der vorstehenden Ansprüche, dadurch gekennzeichnet, dass die Wände, die den unteren Teil jeder Zelle (2) bilden, simultane photometrische Analysen ermöglichen.

7. Vorrichtung zur Simultananalyse nach einem der vorstehenden Ansprüche, dadurch gekennzeichnet, dass der Rotor durch Übereinandersetzen zweier koaxialer Teile (1A, 1B) erhalten wird, deren miteinander in Kontakt stehende periphere Ränder direkt die peripheren Öffnungen (7) der Zellen bilden.

8. Vorrichtung zur Simultananalyse nach einem der vorstehenden Ansprüche, dadurch gekennzeichnet, dass der Haftträger aus mindestens einer Kugel (3) besteht.

9. Vorrichtung zur Simultananalyse nach Anspruch 8, dadurch gekennzeichnet, dass, wenn der Träger aus einer Vielzahl von Kugeln (3') sehr geringer Abmessungen besteht, eine Sperre (20) in jeder Zelle gegenüber der peripheren Ausguss-öffnung (7) angeordnet ist, welche die Kugeln (3') während des Schleuderns zurückhält.

10. Vorrichtung zur Simultananalyse nach Anspruch 9, dadurch gekennzeichnet, dass die Sperre (20) vom Molekularfiltertyp ist.

11. Vorrichtung zur Simultananalyse nach einem der Ansprüche 9 und 10, dadurch gekennzeichnet, dass die Kugeln (3') einen Durchmesser von zwischen 10 und 20 µm besitzen.

12. Vorrichtung zur Simultananalyse nach einem der Ansprüche 1 bis 7, dadurch gekennzeichnet, dass der Haftträger (3'') aus der Wand des unteren Teils jeder Zelle (2) besteht.

13. Vorrichtung zur Simultananalyse nach einem der Ansprüche 8 bis 11, dadurch gekennzeichnet, dass der Boden (9) jeder Zelle (2) eine allgemein geneigte Form aufweist, die so bestimmt ist, dass einerseits der untere Teil der Zelle ein ausreichendes Volumen aufweist, um die Haftträger und die sie bedeckende Menge an Reagenzstoff aufzunehmen, und dass anderseits der Träger im wesentlichen bis zur Höhe der peripheren Öffnung (7) während des Schleuderns geführt wird.

14. Vorrichtung zur Simultananalyse nach einem der Ansprüche 1 bis 12, dadurch gekennzeichnet, dass die inneren (21) und äusseren Seiten (22) der Zellen im wesentlichen parallel sind, wobei die Aussenseite (22) ausserdem einen seitlichen Überlauf (23) aufweist, der in der Ausguss-öffnung (7) endet und ein vollständiges Ausleeren der Waschflüssigkeit während des Schleuderns ermöglicht.

15. Vorrichtung zur Simultananalyse nach Anspruch 14, dadurch gekennzeichnet, dass der Überlauf (23) am hinteren Teil der Zelle (2) in bezug auf die Rotationsrichtung angeordnet ist.

# FIG.1

# FIG.2

8    8    4

1A

3

1B    10    14

2    9    2    13

# FIG.3

2

1    8

5

5

8

2    14

4

0 027 986

# FIG.4A

# FIG.4B

# FIG.4C

13

0 027 986

# FIG.5A

# FIG.5B

15

# FIG.6

# FIG.7